# EUROPEAN PATENT SPECIFICATION

(11) **EP 1 502 908 B1**
(45) Date of publication and mention of the grant of the patent: **12.09.2007**
(21) Application number: 04017173.8
(22) Date of filing: 21.07.2004
(51) Int. Cl.: C07C 43/225, C07D 317/46

(54) **Process for fluorurating ethers**
Verfahren zu Fluorierung von Äthern
Procédé de fluoration d' éthers.

(30) Priority: 30.07.2003 IT mi20031563
(43) Date of publication of application: 02.02.2005
(73) Proprietor: MITENI S.p.A., 20138 Milan (IT)
(72) Inventor: Biasetton, Silvia, 35100 Padova (IT); Marinello, Franco, 36078 Valdagno (VI) (IT)
(74) Representative: Trupiano, Federica

(56) References cited:
- EP-A- 0 567 847
- EP-A- 0 590 459
- EP-A- 0 759 433

## Description

The subject-matter of the present invention is a new process for the preparation of fluorinated derivatives of aromatic ethers. In particular, the invention relates to a process for fluorination of aromatic ethers in the alpha position on the side chain.

The fluorination of ethers in the alpha position is generally performed by exchange of halogen starting from the corresponding chlorinated derivatives.

EP 567847 describes the chlorination of ethers in the alpha position using 4-chloro-benzotrifluoride as solvent, whilst EP 567848 describes the chlorination of 1,3-benzodioxol in defined conditions of simultaneous metering of the reagents and of temperature. Said chlorinated products are then isolated and used in a reaction of fluorination by exchange of halogen, a reaction which according to the known art is performed in the absence of solvents, to obtain the corresponding fluorinated derivatives in the positions referred to above.

The isolation of the intermediate chlorinated derivatives represents a delicate step both because the isolation itself notoriously implies a natural limit of yield and on account of the sensitivity to humidity and the tendency towards hydrolysis of some of these derivatives, in particular those of the family of the benzodioxols.

The subsequent fluorination can be conducted in the absence of solvent. In the case, however, wherein it is necessary to have an accurate control of the exothermicity of the system, the absence of solvent can represent a limit.

There has now been found an original process for the preparation of fluorinated derivatives of ethers that enables the drawbacks of the prior art to be overcome, as well as advantageously enabling excellent yields to be obtained, and is technologically implementable at an industrial level in an altogether convenient way.

In fact, it has now been found that it is advantageous to fluorinate ethers on carbon in the alpha position with respect to oxygen, in particular benzodioxol and anisole, by means of a reaction that envisages a step of chlorination and a step of fluorination in succession, in the same solvent and without isolation and purification of the intermediate chlorinated derivative.

Thus, according to one of its aspects, the invention relates to a process for the preparation of the compounds of formula (I) 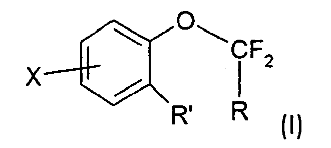 wherein R represents a fluorine atom, R' represents a hydrogen atom or R and R' together are an oxygen atom, and X represents a hydrogen, chlorine or fluorine atom, said process comprising:
a) reacting a compound of formula (II) 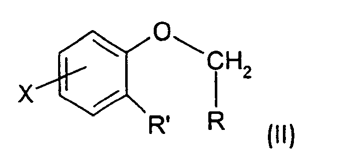 wherein R and R' both represent a hydrogen atom or R and R' together are an oxygen atom, and X is as defined above,
   with gaseous chlorine and in the presence of radical initiators, such as for example UV light, in a solvent chosen among dichloro-benzotrifluorides and their mixtures, in particular 3,4-dichloro-benzotrifluoride, 2,4-dichloro-benzodifluoride;
b) adding hydrofluoric acid to the reaction mixture without isolation of the product obtained in step (a); and
c) isolating the product of formula (I) thus obtained.

According to a preferred aspect of the present invention, X is a hydrogen atom.

According to a particularly preferred aspect, the invention relates to the synthesis of the compound of formula (I') below: 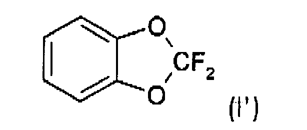 starting from the corresponding following starting product of formula (II'):

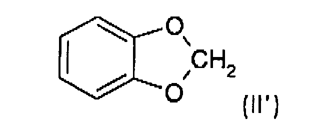

In step (a) of the process of the invention, the chlorination of the compound of formula (I) is carried out according to the usual techniques of radical chlorination, preferably at a temperature of between 130°C and the reflux temperature, advantageously at the reflux temperature of the reaction mixture. In the case wherein the reaction is conducted at the reflux temperature, it will be noted that this varies progressively according to the variation of composition of the mixture.

The solvents indicated previously can also be used, optionally mixed together, 3,4-dichloro-benzotrifluoride and 2,4-dichlorobenzotrifluoride being particularly preferred solvents.

The use of the solvent chosen among the dichloro-benzotrifluorides enables an unexpected improvement in the progress of the reaction as compared to the use of more widely employed solvents, such as, for example, benzotrifluoride and mono-chlorobenzotrifluoride optionally used in this type of reaction, as described in the patents cited previously. This fact may be due to the higher boiling point of dichloro-benzotrifluorides with respect to that of benzotrifluoride and chloro-benzotrifluoride. It was in fact noted that the fact of being able to conduct chlorination at a higher temperature helps to limit significantly the formation of by-products, thus favouring a better yield in the chlorinated derivative in the alpha position on the side chain.

The concentration of the mixture to be subjected to the reaction of chlorination varies within a wide range; for example, it may vary between 20 wt% and 60 wt% of substrate with respect to the solvent and preferably is approximately 50 wt%.

The reaction of step (a) is normally completed in a few hours, for example from 2 to 8 hours. The course of the reaction can be followed by the technician according to customary analytical procedures, such as, for example, gas-chromatography .

At the end of the reaction of step (a), fluorination of the reaction mixture is carried out without isolation of the intermediate derivative and without further addition or separation of solvent.

In practice, to proceed with the reaction of fluorination of step (b), it is sufficient to introduce the mixture of the reaction of step (a) into an appropriate reactor and then add hydrofluoric acid.

Preferably, the reaction is conducted at a temperature of between - 10°C and room temperature, and in particular, when the aim is to obtain the compound of formula (I), wherein R and R' together are an oxygen atom, for example to obtain the compound (I'), cooling the reaction mixture to approximately 0-5°C so as to contain the possible exothermicity of this halogen-exchange reaction.

The amount of hydrofluoric acid added is at least stoichiometric, even though an excess of hydrofluoric acid is preferable; according to an advantageous aspect of the invention, a molar excess of hydrofluoric acid is used of at least 100% with respect to the theoretical amount of chlorinated intermediate, or with respect to the amount of starting product of formula (II).

The fact of operating with an excess of hydrofluoric acid does not only ensure always obtaining the maximum conversion but also renders the metering operations particularly convenient and enables compensation of the inevitable losses of hydrofluoric acid, which are concomitant with the operations of removal of the hydrochloric acid that forms during the reaction of fluorination.

In fact, the fluorination reaction is preferably conducted in a reactor by adding the hydrofluoric acid in portions and verifying the development of gaseous hydrochloric acid, which is advantageously removed by means of venting prior to addition of the subsequent portion of hydrofluoric acid.

The reaction is concluded when hydrochloric acid is no longer produced, this being confirmed by the stabilization of the pressure inside the reactor.

The use of solvents as in the preceding step (a) of the process also for the subsequent fluorination step (b) leads to a substantial advantage as compared to the reaction conducted in the absence of solvent in so far as it enables a better control of the exothermicity of reaction thanks to the effect of thermal flywheel, which distributes the heat produced and hence enables a faster cooling of the mixture.

The end product can finally be recovered according to known techniques.

According to a preferred embodiment of the invention, when the reaction of step (b) is completed, the temperature of the reactor is raised to evaporate the hydrofluoric acid in excess. For example, there may be envisaged an increase in the temperature of up to approximately 30-50°C to evaporate the excess hydrofluoric acid, which will then be recovered to be reused if so desired or necessary.

Once the reaction is concluded with removal of the excess acid, the end product can be directly isolated according to known techniques.

According to a particularly advantageous embodiment, the end product of formula (I) is isolated by distillation.

Through the process of the invention, the product of formula (I) is obtained with global yields higher than 80% and with yields of the individual passages (chlorination, fluorination, distillation) higher than 90% and presents an extremely high degree of purity, exceeding 99%, for instance even reaching 99.9%, which can be verified by means of gas chromatography.

The process according to the invention hence makes it possible to prepare the fluorinated derivatives on the aliphatic carbon of benzodioxol and of anisole through a synthesis which is original, reliable from the industrial standpoint, free from any significant production of reaction by-products, without isolation of the intermediate chlorinated derivative (which is readily subject to degradation), and hence requires a single final rectification to obtain the desired product with high yields.

The ensuing examples illustrate the invention without limiting it in any way.

### EXAMPLE 1

### Preparation of 2,2-difluoro-1,3-benzodioxol

### (a) Preparation of 2,2-dichloro-1,3-benzodioxol

A 5-litre lined reactor equipped with a UV lamp, a bubbler for chlorine, and a water-circulation reflux condenser, was charged with 1685 g of 1,3-benzodioxol and 1685 g of 3,4-dichloro-benzotrifluoride. The mixture was heated under reflux in a nitrogen stream. The 50-W UV lamp was then turned on, and bubbling of the chlorine was started. Completion of the reaction was verified through gas-chromatography. There were obtained 4161 g of a mixture of 2,2-dichloro-1,3-benzodioxol and 3,4-dichloro-benzotrifluoride. The chromatographic titre of 2,2-dichloro-1,3-benzodioxol (excluding the solvent) was 98 %.

### (b) Preparation of 2,2-difluoro-1,3-benzodioxol

1040g of mixture obtained with the reaction of step (a) were introduced into a 3-litre steel reactor, and the system was cooled to 0-3°C under stirring. Metering of the hydrofluoric acid in portions of 70 ml each for a total amount of 280 g was started. During addition, the vent of the reactor was left open; at the end of the addition it was re-closed, and the internal pressure was allowed to rise to 4-5 atm. After approximately 15 minutes following upon each addition, the hydrochloric acid formed was vented, and a fresh addition of hydrofluoric acid was made. At the end of this addition, the reaction mixture was heated to approximately 50°C to remove any excess of hydrofluoric acid. The product was purified by distillation, first eliminating the residual hydrofluoric acid, and rectification was performed to obtain the product of the titre.

There were obtained 1805 g of the product with a purity of 99.9 % (GC). The global yield of chlorination, fluorination and distillation was 83%.

### EXAMPLE 2

### Preparation of 2,2-difluoro-1,3-benzodioxol

### (a) Preparation of 2,2-dichloro-1,3-benzodioxol

A 1-litre reactor equipped with UV lamp, bubbler for chlorine, and water-circulation reflux condenser, was charged with 337 g of 1,3-benzodioxol and 337 g of 2,4-dichloro-benzotrifluoride. The mixture was heated under reflux in a nitrogen stream. The 50-W UV lamp was then turned on, and bubbling of the chlorine was started. Completion of the reaction was verified through gas-chromatography. The duration of the reaction was 4 hours. There were obtained 846 g of mixture of 2,2-dichloro-1,3-benzodioxol and 2,4-dichloro-benzotrifluoride. The chromatographic titre of 2,2-dichloro-1,3-benzodioxol (excluding the solvent) was 99 %.

### (b) Preparation of 2,2-difluoro-1,3-benzodioxol

846 g of mixture obtained with the reaction of step (a) were introduced in a 3-litre steel reactor, and the system was cooled to 0-4°C under stirring. Metering of the hydrofluoric acid in portions (70 ml, 80 ml, 80 ml) was started for a total amount of 230 g. During the addition, the vent of the reactor was left open; at the end of the addition, it was re-closed, and the internal pressure was allowed to rise to 4-5 atm. After approximately 15 minutes following upon each addition, the hydrochloric acid formed was vented, and a new addition of hydrofluoric acid was performed. At the end of this operation, the reaction mixture was heated to approximately 50°C to remove the excess hydrofluoric acid. The product was purified by distillation, first eliminating the residual hydrofluoric acid, and rectification was then performed to obtain the product of the titre.

There were obtained 373 g, with a purity of 99.9 % (GC). The global yield of chlorination, fluorination and distillation was 85%.

### EXAMPLE 3

### Preparation of 2,2-difluoro-1,3-benzodioxol

### (a) Preparation of 2,2-dichloro-1,3-benzodioxol

A 1-litre reactor equipped with UV lamp, bubbler for chlorine, and water-circulation reflux condenser, was charged with 337 g of 1,3-benzodioxol and 337 g of 3,4-dichloro-benzotrifluoride. The mixture was heated to 135-140°C in a nitrogen stream. The 50-W UV lamp was then turned on, and bubbling of the chlorine was started. Completion of the reaction was verified through gas-chromatography. 879 g of mixture of 2,2-dichloro-1,3-benzodioxol and 3,4-dichloro-benzotrifluoride were obtained.

The chromatographic titre of 2,2-dichloro-1,3-benzodioxol (excluding the solvent) was 96 %.

Then step (b) was performed according to what is described in the foregoing examples.

## Claims

1. A process for the preparation of a compound of formula (I) 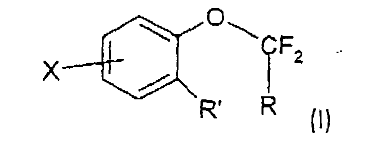 wherein R represents a fluorine atom, R' represents a hydrogen atom or R and R' together are an oxygen atom, and X represents an atom of hydrogen, chlorine or fluorine, said process being **characterized by**:
a) reacting a compound of formula (II) 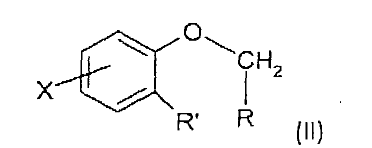 wherein R and R' both represent a hydrogen atom or R and R' together are an oxygen atom, and X represents an atom of hydrogen, chlorine or fluorine,
and gaseous chlorine and in the presence of a radical initiator, in a solvent chosen among a dichloro-benzotrifluoride and their mixtures without isolation of the product obtained in step (a); and
b) adding hydrofluoric acid to the reaction mixture; and
c) isolating the product of formula (I) thus obtained.

2. The process according to Claim 1, **characterized in that** X is a hydrogen atom.

3. The process according to Claim 2, **characterized in that** the starting product of formula (II) is 1,3-benzodioxol of formula (II') 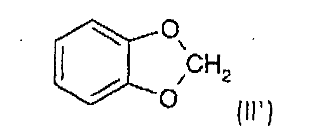 and the product of formula (I) is 2,2-difluoro-1,3-benzodioxol (I')

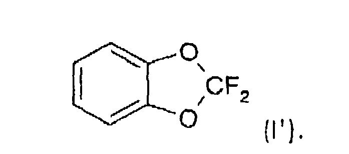

4. The process according to Claims 1 to 3, **characterized in that** step (a) is performed at a temperature of between 130°C and the reflux temperature of the reaction mixture.

5. The process according to Claim 4, **characterized in that** step (a) is performed at the reflux temperature of the reaction mixture.

6. The process according to Claims 1 to 3, **characterized in that** in step (a) UV light is used as radical initiator.

7. The process according to Claims 1 to 3, **characterized in that** the solvent is 3,4-dichloro-benzotrifluoride.

8. The process according to Claims 1 to 3, **characterized in that** the solvent is 2,4-dichloro-benzotrifluoride.

9. The process according to Claims 1 to 3, **characterized in that** step (b) is performed at a temperature of between -10°C and room temperature.

10. The process according to Claim 3, **characterized in that** step (b) is performed at a temperature of between 0°C and +5°C.

11. The process according to Claims 1 to 3, **characterized in that** in step (b) the hydrofluoric acid is added in portions.

12. The process according to Claims 1 to 3, **characterized in that** in step (b) the hydrofluoric acid is added in an at least stoichiometric amount with respect to the starting product.

13. The process according to Claim 12, **characterized in that** in step (b) the hydrofluoric acid is added in a molar excess of at least 100% with respect to the starting product.

14. The process according to Claims 1 to 3, **characterized in that** in step (c) the product is isolated by distillation.

15. The process according to Claim 14, **characterized in that** in step (c) prior to isolation of the product of formula (I) the excess hydrofluoric acid is removed.

## Patentansprüche

1. Verfahren zur Herstellung einer Verbindung der Formel (I) 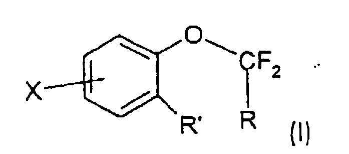 worin R ein Fluor-Atom bedeutet, R' ein Wasserstoff-Atom bedeutet oder R und R' zusammen ein Sauerstoff-Atom sind, und X ein Wasserstoff-, Chlor- oder Fluor-Atom bedeutet, **gekennzeichnet durch**:
a) Umsetzung einer Verbindung der Formel (II) 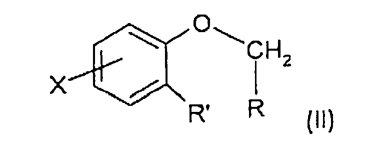 worin sowohl R als auch R' ein Wasserstoff-Atom bedeuten, oder R und R' zusammen ein Sauerstoff-Atom sind, und X ein Wasserstoff-, Chlor- oder Fluor-Atom bedeutet,
mit gasförmigem Chlor in Gegenwart eines Radikalstarters in einem Lösungsmittel, das ausgewählt ist aus Dichlorbenzotrifluorid und Mischungen davon;
b) Zugabe von Fluorwasserstoffsäure zur Reaktionsmischung ohne Isolierung des in Schritt a) erhaltenen Produkts; und
c) Isolierung des so erhaltenen Produkts der Formel (I).

2. Verfahren nach Anspruch 1, **dadurch gekennzeichnet, dass** X ein Wasserstoff-Atom ist.

3. Verfahren nach Anspruch 2, **dadurch gekennzeichnet, dass** das Ausgangsmaterial der Formel (II) das 1,3-Benzodioxol der Formel (II') ist, 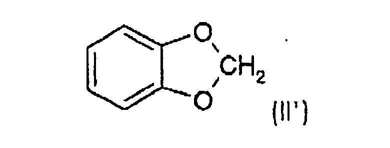 und das Produkt der Formel (II) das 2,2-Difluor-1,3-benzodioxol (I') ist

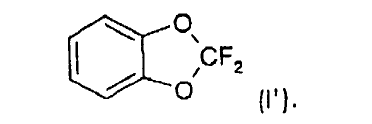

4. Verfahren nach den Ansprüchen 1 bis 3, **dadurch gekennzeichnet, dass** der Schritt a) bei einer Temperatur zwischen 130°C und der Rückflusstemperatur der Reaktionsmischung durchgeführt wird.

5. Verfahren nach Anspruch 4, **dadurch gekennzeichnet, dass** der Schritt a) bei der Rückflusstemperatur der Reaktionsmischung durchgeführt wird.

6. Verfahren nach den Ansprüchen 1 bis 3, **dadurch gekennzeichnet, dass** UV-Licht bei Schritt a) als Radikalstarter verwendet wird.

7. Verfahren nach den Ansprüchen 1 bis 3, **dadurch gekennzeichnet, dass** das Lösungsmittel 3,4-Dichlorbenzotrifluorid ist.

8. Verfahren nach den Ansprüchen 1 bis 3, **dadurch gekennzeichnet, dass** das Lösungsmittel 2,4-Dichlorbenzotrifluorid ist.

9. Verfahren nach den Ansprüchen 1 bis 3, **dadurch gekennzeichnet, dass** der Schritt b) bei einer Temperatur zwischen -10°C und Raumtemperatur durchgeführt wird.

10. Verfahren nach Anspruch 3, **dadurch gekennzeichnet, dass** der Schritt b) bei einer Temperatur zwischen 0°C und +5°C durchgeführt wird.

11. Verfahren nach den Ansprüchen 1 bis 3, **dadurch gekennzeichnet, dass** die Fluorwasserstoffsäure in Schritt b) portionsweise zugesetzt wird.

12. Verfahren nach den Ansprüchen 1 bis 3, **dadurch gekennzeichnet, dass** die Fluorwasserstoffsäure in Schritt b) zumindest in stöchiometrischer Menge gegenüber dem Ausgangsmaterial zugesetzt wird.

13. Verfahren nach Anspruch 12, **dadurch gekennzeichnet, dass** die Fluorwasserstoffsäure in Schritt b) in einem molaren Überschuss von wenigstens 100% gegenüber dem Ausgangsmaterial zugesetzt wird.

14. Verfahren nach den Ansprüchen 1 bis 3, **dadurch gekennzeichnet, dass** das Produkt in Schritt c) durch Destillation isoliert wird.

15. Verfahren nach Anspruch 14, **dadurch gekennzeichnet, dass** in Schritt c) vor der Isolierung des Produkts der Formel (1) die überschüssige Fluorwasserstoffsäure entfernt wird.

## Revendications

1. Procédé de préparation d'un composé de formule (I) : 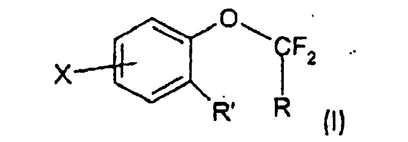 dans laquelle R représente un atome de fluor, R' représente un atome d'hydrogène ou bien R et R' sont ensemble un atome d'oxygène, et X représente un atome d'hydrogène, de chlore ou de fluor, ledit procédé étant **caractérisé par** :
(a) la réaction d'un composé de formule (II) : 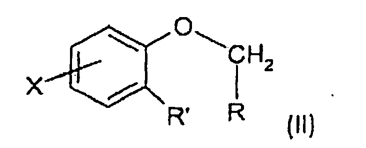 dans laquelle R et R' représentent tous les deux un atome d'hydrogène ou bien R et R' sont ensemble un atome d'oxygène et X représente un atome d'hydrogène, de chlore ou de fluor,
et de chlore gazeux et en présence d'un initiateur de radicaux, dans un solvant choisi parmi un dichlorobenzotrifluorure et leurs mélanges ;
(b) l'addition d'acide fluorhydrique au mélange réactionnel sans isolation du produit obtenu dans l'étape (a) ; et
(c) l'isolation du produit de formule (I) ainsi obtenu.

2. Procédé selon la revendication 1, **caractérisé en ce que** X est un atome d'hydrogène.

3. Procédé selon la revendication 2, **caractérisé en ce que** le produit de départ de formule (II) est le 1,3-benzodioxol de formule (II') : 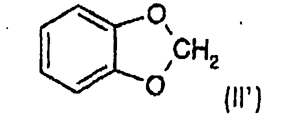 et que le produit de formule (I) est le 2,2-difluoro,1,3-benzodioxol (1') :

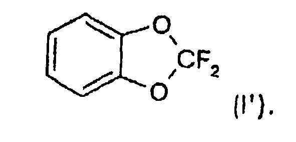

4. Procédé selon les revendications 1 à 3, **caractérisé en ce que** l'étape (a) est réalisée à une température comprise entre 130°C et la température de reflux du mélange réactionnel.

5. Procédé selon la revendication 4, **caractérisé en ce que** l'étape (a) est réalisée à la température de reflux du mélange réactionnel.

6. Procédé selon les revendications 1 à 3, **caractérisé en ce que** dans l'étape (a), une lumière UV est utilisée en tant qu'initiateur de radicaux.

7. Procédé selon les revendications 1 à 3, **caractérisé en ce que** le solvant est le 3,4-dichloro-benzotrifluorure.

8. Procédé selon les revendications 1 à 3, **caractérisé en ce que** le solvant est le 2,4-dichloro-benzotrifluorure.

9. Procédé selon les revendications 1 à 3, **caractérisé en ce que** l'étape (b) est réalisée à une température comprise entre -10°C et la température ambiante.

10. Procédé selon la revendication 3, **caractérisé en ce que** l'étape (b) est réalisée à une température comprise entre 0°C et +5°C.

11. Procédé selon les revendications 1 à 3, **caractérisé en ce que** dans l'étape (b), l'acide fluorhydrique est ajouté par parties.

12. Procédé selon les revendications 1 à 3, **caractérisé en ce que** dans l'étape (b), l'acide fluorhydrique est ajouté en une quantité au moins stoechiométrique par rapport au produit de départ.

13. Procédé selon la revendication 12, **caractérisé en ce que** dans l'étape (b), l'acide fluorhydrique est ajouté en un excès molaire d'au moins 100% par rapport au produit de départ.

14. Procédé selon les revendications 1 à 3, **caractérisé en ce que** dans l'étape (c), le produit est isolé par distillation.

15. Procédé selon la revendication 14, **caractérisé en ce que** dans l'étape (c) avant l'isolation du produit de formule (I) l'acide fluorhydrique en excès est éliminé.
